# EUROPÄISCHE PATENTSCHRIFT

(11) **EP 0 310 883 B1**
(45) Veröffentlichungstag und Bekanntmachung des Hinweises auf die Patenterteilung: **17.03.1993**
(21) Anmeldenummer: 88115643.4
(22) Anmeldetag: 23.09.1988
(51) Int. Cl.: C12P 7/06, C12M 1/12

(54) **Verfahren zur kontinuierlichen fermentativen Erzeugung von niederen aliphatischen Alkoholen oder organischen Lösungsmitteln**
Process for the continuous production of low aliphatic alcohols or organic solvents
Procédé pour la préparation en continu d'alcools aliphatiques inférieurs ou de solvants organiques

(30) Priorität: 09.10.1987 DE 3734124
(43) Veröffentlichungstag der Anmeldung: 12.04.1989
(73) Patentinhaber: Starcosa GmbH, D-38122 Braunschweig (DE)
(72) Erfinder: Tegtmeier, Uwe, Dr., D-3341 Wittmar (DE)
(74) Vertreter: Einsel, Martin Dipl.-Phys.

(56) Entgegenhaltungen:
- US-A- 4 310 629
- US-A- 4 385 118

## Beschreibung

Die Erfindung betrifft ein Verfahren zur kontinuierlichen fermentativen Erzeugung von niederen aliphatischen Alkoholen oder organischen Lösungsmitteln, insbesondere von Äthanol, aus zuckerhaltigen Substraten, wie Zuckerrübenmelasse, bei dem das Substrat in zwei aufeinanderfolgenden Fermentationsstufen der Einwirkung von alkoholbildenden Mikroorganismen, insbesondere Hefe, bei unsteriler Prozeßführung ausgesetzt wird.

Die fermentative Erzeugung von niederen aliphatischen Alkoholen sowie von organischen Lösungsmitteln gewinnt seit einiger Zeit zunehmend an Bedeutung. Zum einen wird hierdurch ein Weg aufgezeichnet, bei der künftigen Verknappung an fossilen Energieträgern einen Ersatz auf der Basis nachwachsender Rohstoffe zu schaffen, wie dieses insbesondere bei der Produktion von Äthanol, Butanol und Aceton der Fall ist, zum anderen lassen sich auf fermentativem Wege beständigere Alkohole für die chemische Weiterverarbeitung produzieren, als dieses auf Basis Erdöl geschehen kann. Ein Beispiel hierfür stellt 2,3-Butandiol dar, welches durch katalytische Dehydrierung auf chemischem Wege nicht wirtschaftlich gewonnen werden kann.

Insbesondere Äthanol ist ein Grundstoff für die zukünftige Verwendung als Basiskomponente weiterführender chemischer Reaktionen, wie auch als flüssiger Energieträger und somit Treibstoffzusatz.

Im Zuge fortlaufender Automatisierung von Produktionsprozessen werden zunehmend kontinuierliche Verfahren gefordert. Im Rahmen der Produktion von Äthanol wie auch anderer organischer Lösungsmittel und niederer aliphatischer Alkohole stellt die Fermentation den bislang weitestgehend absatzweise geführten Prozeßteilschritt dar. Eine kontinuierliche Prozeßführung würde hier den Gesamt-Produktionsbetrieb einer kontinuierlichen Arbeitsweise zuführen, so daß Prozeßüberwachung arbeitsextensiver gestaltet werden könnte.

Für die Weiterverarbeitung dieser auf fermentativer Basis produzierten chemischen Verbindungen ist es wichtig, in der Fermentation eine hohe Produktkonzentration zu erzielen, um diese weiterführenden Prozeßschritte mit einem möglichst geringen Energieaufwand betreiben zu können.

Ferner wird angestrebt, daß kontinuierliche Fermentationsverfahren stabil ablaufen, d.h. bei biologischen Prozessen sollen Infektionen, also Kontaminationen mit Fremdmikroorganismen möglichst nicht entstehen, oder die Konzentration dieser Fremdmikroorganismen muß so gering gehalten werden können, daß Nebenproduktbildungen den Prozeß nicht stören.

Die bekannten kontinuierlichen Fermentationsverfahren, insbesondere zur Produktion von Äthanol, wie in der US-A 43 85 118 beschrieben, erfüllen diese Ansprüche nicht. Dieses Verfahren geht von sterilen Zuckerlösungen aus und erfordert eine sterile Arbeitsweise. Es ist somit bei diesen Verfahren notwendig, eine energieaufwendige in-line Sterilisation oder Rohstoffsterilisation vorzunehmen, um Infektionen zu vermeiden. Die für eine mikrobiologisch stabile Betriebsweise benötigten Biomassenkonzentrationen können nicht eingestellt werden, da die Führung der Biomasse im Prozeß die Abtrennung mittels eines Separators erfordert. Die Biomassenabtrennung über Separatoren versagt völlig, wenn als produzierende Mikroorganismen Bakterien eingesetzt werden.

Ein kontinuierliches Fermentationsverfahren, welches mit entsprechend hohen Zellkonzentrationen arbeitet und anstelle von Separatoren Membranen zur internen Zellaufkonzentrierung und Zellrückhaltung einsetzt, ist in der EU-Anmeldung 85 103 044.6 beschrieben. Hierbei wird in einem in seiner Betriebsweise als Membranreaktor funktionierenden Fermenter eine Biomassenkonzentration eingestellt, die aufgrund der dadurch entstehenden Subnstratkonkurrenz gegenüber Fremdmikroorganismen zu einer weitestgehend stabilen Arbeitsweise befähigt.

Das vorgenannte Verfahren hat den Nachteil, daß bei Prozessen, die mit hoher Endproduktkonzentration und somit starken Streßbedingungen für den arbeitenden Mikroorganismus ablaufen sollen, eine ausreichende Neubildung an Biomasse und Erhaltung der Aktivität über längere Zeiten in dem aus einem Fermenter bestehenden System nicht gewährleistet ist.

Der Erfindung liegt die Aufgabe zugrunde, ein Verfahren der einleitend genannten Art so auszubilden, daß es eine kontinuierliche Fermentation zur Erzeugung der genannten niederen aliphatischen Alkohole oder organischen Lösungsmittel, vorzugsweise von Äthanol, ermöglicht, bei der eine hohe aktive Zellkonzentration des produzierenden Mikroorganismusses aufrechterhalten, eine hohe Produktkonzentration sowie eine hohe Produktivität bei unsteriler Prozeßführung erreicht werden.

Gelöst wird die vorstehende Aufgabe erfindungsgemäß durch die im kennzeichnenden Teil des Hauptanspruches aufgeführten Merkmale.

Durch das neue Verfahren wird es möglich, das mittlere Zellalter der Population relativ hoch einzustellen, wodurch die Verluste an Biomasse niedrig gehalten werden können. Dieses führt zu einer Reduzierung von Ausbeuteverlusten.

Die Aktivierungsstufe wird derart betrieben, daß zum einen hohe Wachstumsraten des produzierenden Mikroorganismusses erreicht werden, zum anderen jedoch die Milieubedingungen für Fremdmikroorganismen äußerst ungünstig sind. Insbesondere bei der Äthanolproduktion mittels Hefen kann dadurch der Überwuchs durch Fremdhefe verhindert werden. Ein wichtiges Selektivitätskriterium stellt hierbei der osmotische Druck des Mediums dar. Äthanol produzierende Hefen sind fakultativ äthanoltoleranter als reine Wuchshefen. Durch Überführung eines Stromes aus der Aktivierungsstufe in die Produktstufe wird der Produktstufe kontinuierlich ein hochaktiver Mikroorganismenstrom mit niederem Zellalter zugeführt. Dies bewirkt, daß in der Produktstufe die Mediumsbedingungen derart gewählt werden können, daß enorme Streßbedingungen für osmosensitive und äthanolsensitive Mikroorganismen entstehen, ohne die produzierende Biomasse in ihrer Aktivität signifikant einzuschränken. Daraus folgt, daß eine konstant hohe Biomassenkonzentration über einen langen Arbeitszeitraum in der Produktstufe aufrechterhalten werden kann. Dabei ist es möglich, die mittlere Verweilzeit der Zellen zu verlängern, was zu erhöhten Produktausbeuten führt.

Eine Gewinnung der Permeatströme, deren Größe zur Einstellung der Milieubedingungen in der Aktivierungsstufe und in der Produktstufe dient, wird mittels Mikrofiltrationsmodulen vorgenommen. Um bestmögliche Betriebsergebnisse zu erzielen, ist es sinnvoll, Hollow-fiber Module zu verwenden. Bei Einsatz anderer Bauarten steigen entweder die Produktionskosten durch erhöhte Retentatströme stark an, oder es entstehen derartig unzulässige Schergefälle beim Durchströmen der Membranen (z.B. Wickelmodule), daß eine mechanische Schädigung der Biomasse auftritt.

Durch die Einstellung des Ablaufes aus der Produktstufe wird der Gehalt an Gesamttrockensubstanz im System begrenzt und es wird der zur Einstellung des mittleren Zellalters notwendige Biomassestrom aus dem System entfernt. In diesem Ablaufstrom befinden sich sämtliche Komponenten, die als nichtlösliche Bestandteile des Mediums hinsichtlich ihrer Partikelgröße über den maximalen Porengrößen der Mikrofiltrationsmembranen liegen. Hierzu zählen insbesondere flockoliertes Eiweiß, Bestandteile autolisierter Zellen und Polysaccharide.

Besonders günstig ist es, einen Teilstrom des Retentates aus der Produktstufe in die Aktivierungsstufe zurückzuführen. Dadurch erhöht sich ebenfalls der aus der Aktivierungsstufe in die Produktstufe zu leitende biomassenhaltige Strom. Insgesamt wird damit eine kürzere Verweilzeit der Biomasse in der Produktstufe erreicht und die Biomasse erfährt jeweils zwischenzeitlich bei verminderten Streßbedingungen in der Aktivierungsstufe eine Aktivierung ihrer Atmungsenzyme. Auf diese Weise wird der ATP-Gehalt in der Zelle erhöht.

Diese Verfahrensweise ist zweckmäßig anzuwenden, wenn Mikroorganismen mit sehr niedrigen Wachstumsraten unter den zuvor beschriebenen hohen Streßbedingungen in der Produktionsstufe eingesetzt werden sollen.

Eine weitere zweckmäßige Maßnahme ist im Anspruch 3 angegeben.

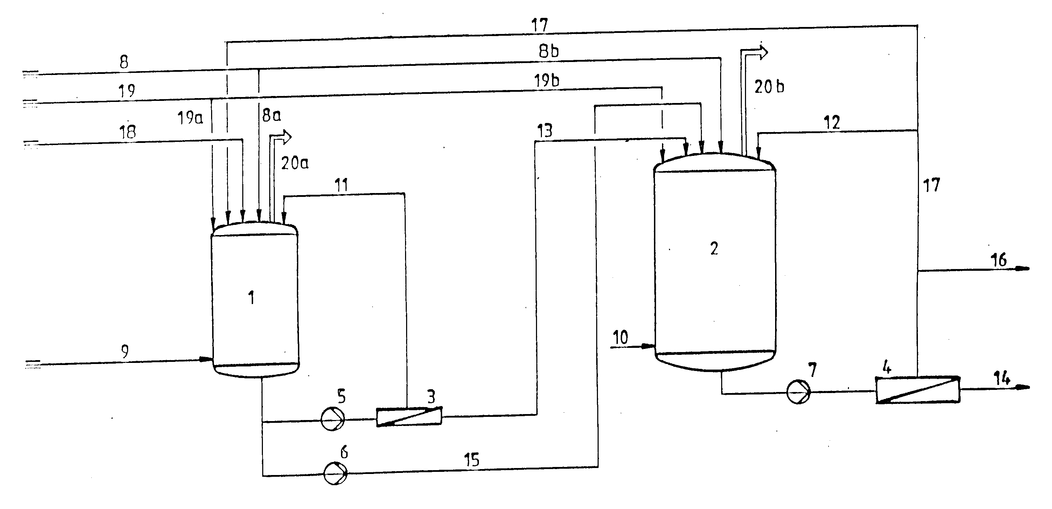

Die beigefügte Zeichnung gibt ein prinzipielles Fließschema für das Verfahren gemäß der Erfindung wieder.

Die Aktivierungsstufe 1 wird sowohl wie die Produktstufe 2 über die aus der Sammelleitung 8 abgezweigten Zuführungen 8a,8b mit Substrat beschickt. Die Zuführung an weiteren Nähr- und Wuchsstoffen geschieht lediglich zur Aktivierungsstufe 1 über Leitung 18. Die Begasung beider Fermenter zwecks Zuführung von Sauerstoff erfolgt über Leitungen 9 zur Aktivierungsstufe 1 und Leitung 10 zur Produktstufe 2.

Die Erzeugung des Permeatstromes 13 der Aktivierungsstufe 1 geschieht durch Umpumpen eines Retentatstromes 11 mittels Pumpe 5 durch den Mikrofiltrationsmodul 3. Der biomassehaltige Ablaufstrom 15 der Aktivierungsstufe 1 zum Produktfermenter 2 wird durch Einsatz der Pumpe 6 vorgenommen.

Aus der Produktstufe 2 wird ein Permeatstrom 14 gewonnen, indem mittels Pumpe 7 durch den Mikrofiltrationsmodul 4 ein Retentatstrom 12 umgepumpt wird. Die Größe des hier abgezogenen Permeatstromes 14 richtet sich überwiegend nach der einzustallenden Produktkonzentration. Als biomassehaltiger Strom der Produktstufe 2 wird ein Teilstrom 16 des Retentatstromes 12 abgeführt.

Zum Ausgleich der Arbeitsvolumina wird Prozeßwasser über die Sammelleitung 19 und Teilzuführungsleitungen 19a und 19b sowohl zur Aktivierungsstufe 1 als auch zur Produktstufe 2 zugeführt.

Die nicht verbrauchten Bestandteile des zwecks Sauerstoffzuführung eingeleiteten Begasungsmediums sowie die reduktionsäquivalenten Mengen an CO₂ verlassen die jeweiligen Fermenter über die Abgasleitungen 20a sowie 20b.

In dem dargestellten Beispiel wird ein Teilstrom des biomassehaltigen Mediums der Produktstufe 2 über die Leitung 17 in die Aktivierungsstufe 1 zurückgeführt.

Die Erfindung wird nachstehend an zwei Beispielen näher erläutert.

### Beispiel 1

Dieses Beispiel beschreibt die kontinuierliche fermentative Erzeugung von Äthanol.

Es wurde ausgehend von einer Reinkultur eine Anzucht der produktbildenden Biomasse in den beiden Fermentern 1 und 2 vorgenommen, wobei der Aktivierungsfermenter 1 ca. 20 Std. nach dem Produktfermenter 2 in Betrieb genommen wurde. Als Mikroorganismusstamm diente hierzu eine handelsübliche Backhefe. Die Anzucht geschah bis zur gewünschten Biomassekonzentration und wurde in möglichst kurzer Zeit unter optimalen Wachstumsbedingungen, d.h. Mediumsbedingungen entsprechend Temperatur = 30°C, pH-Wert = 4,8, lösliche Nichtzuckertrockensubstanz = 4,0 %, Sauerstoffpartialdruck = 2 % sowie ausreichende Nähr- und Wuchsstoffversorgung zum Erreichen hoher Wuchstumsraten durchgeführt.

Als Substrat wurde Rübenzuckermelasse verwendet.

Die den Fermentern zugeführte Melassemenge wurde so eingestellt, daß durch Überdosierung der verwertbaren C-Quelle die gleichzeitige Bildung von Äthanol induziert wurde.

Eine Einstellung der Mediumsbedingungen sowie der Alkoholkonzentration von ca. 2 Vol.% geschah durch die Entnahme von Permeatströmen aus den Filtermodulen 3 und 4. Diese Module waren mit Hollow-fiber Modulen der Firma Enka AG, 5600 Wuppertal, Typ MD 080, TP 2L in Modul 3 und Typ MD 150 CP 2N in Modul 4 ausgerüstet.

Ausgehend von ca. 200 g Hefetrockensubstanz (HTS) der Reinkultur in der Aktivierungsstufe 1 mit einem Arbeitsvolumen von 80 Litern und ca. 400 g Hefetrockensubstanz der Reinkultur in der Produktstufe 2 mit einem Arbeitsvolumen von ca. 400 Litern wurde die angestrebte Konzentration an Biomasse von ca. 50 g HTS/l in der Aktivierungsstufe 1 nach ca. 30 Stunden und 110 g HTS/l in der Produktstufe 2 nach ca. 50 Stunden erreicht. Aufgrund einer linear eingestellten Wachstumsrate betrug das mittlere Zellalter der Population ca. 30 bis 35 Stunden zu diesem Zeitpunkt.

Anschließend wurden sämtliche Parameter so eingestellt, daß die Fermentationsanlage mit maximaler Produktivität und Substratausbeute bei hoher Produktkonzentration betrieben werden konnte.

Eine ausreichende Zellaktivität ließ sich bei einem mittleren Zellalter von ca. 65 Stunden erwarten. Das mittlere Zellalter ist durch die Wahl der Größe des biomassehaltigen Ablaufes 16 der Produktstufe 2 einstellbar und reziprok der mittleren Verweilzeit der Biomasse bei konstanter Biomassekonzentration, da bei kontinuierlichem Betrieb eines Chemostaten die Wachstumsrate (l/h) sich entsprechend der Durchsatzrate D (l/h) also der mittleren Verweilzeit verhält.

Bei den sich in der Produktstufe 2 ergebenden extremen Streßbedingungen für die Hefe findet ein Wachstum in nennenswerter Größe nicht statt. Die mit dem biomassehaltigen Ablauf 16 abgeführte Zellmasse wurde somit durch Zulauf eines biomassehaltigen Stromes 15 aus der Aktivierungsstufe 1 ersetzt.

Da das Wachstum der Mikroorganismen in der Aktivierungsstufe 1 und die Produktbildung in der Produktstufe 2 stattfinden, müssen die Mediumsbedingungen unterschiedlich gewählt werden.

Die Konzentration an Produkt und letztlich auch die Mediumsbedingungen werden durch die Wahl der Größe der Permeatströme 13 und 14 vorgegeben, wobei der Permeatstrom 14 sowie der biomassehaltige Ablauf 16 die produkthaltigen Ablaufströme aus dem Prozeß darstellen.

Der benötigte Sauerstoff wurde mittels Luft eingetragen. Die Mengen an Luft wurden über den aktuellen Sauerstoffpartialdruck geregelt und waren entsprechend den jeweiligen Stofftransportvoraussetzungen variabel.

Die Rübenzuckermelasse hatte einen Gesamtgehalt an fermentierbarem Zucker von 40 Gew.% und Begleitkomponenten in üblicher Größe.

Wuchs- und Nährstoffe wurden entsprechend den in der Backhefeproduktion üblichen Mengen lediglich über Leitung 18 in die Aktivierungsstufe 1 zudosiert.

Die Menge an Prozeßwasser, welches beiden Stufen zulief über Leitungen 19a,19b war eine resultierende Größe, da die Füllstandsregelung der Fermenter dieser Stufen mittels dieser Mengen vorgenommen wurde. Die Wassermengen konnten nicht erfaßt werden, lassen sich aber durch Bilanzierung errechnen.

Die über die Abgasleitungen 20a und 20b abgeführten Abgasmengen setzen sich aus den überschüssigen Begasungsmengen zusammen sowie den jeweiligen reduktionsäquivalenten CO₂-Mengen. Diese Ströme wurden ebenfalls nicht erfaßt, sondern lediglich zur Ausbeuteberechnung bezüglich der hier erhaltenen Produktmengen mit ausreichender Genauigkeit abgeschätzt.

Sämtliche wichtigen wählbaren Stoffströme und Prozeßparameter sind in Tabelle 1 zusammengestellt, die sich im Gleichgewichtszustand einstellenden Mediumsbedingungen und Prozeßparameter in Tabelle 2. Tabelle 3 gibt die aus den gemessenen bzw. eingestellten Größen der Tabellen 1 und 2 errechenbaren, zur Beurteilung des Prozesses wichtigen Verfahrensgrößen wieder.

**Tabelle 1**

| wählbarer Parameter (Bezeichnung laut Zeichnung l) | Wert | Einheit |
|---|---|---|
| Arbeitsvolumen Aktivierungsstufe (l) | 80 | l |
| Arbeitsvolumen Produktstufe (2) | 400 | l |
| Zulauf Substrat (8a) | 7,2 | kg |
| Zulauf Substrat (8b) | 44,0 | kg |
| Produktstufenablauf (16) | 6,15 | l/h |
| Aktivierungsstufenablauf (15) | 8 | l/h |
| Permeatstrom Aktivierungsstufe (13) | 20 | l/h |
| Permeatstrom Produktstufe (14) | 173 | l/h |
| Retentatstrom | | |
| Aktivierungsstufe (11) | 2 | m³/h |
| Retentatstrom Produktstufe (12) | 7 | m³/h |
| Temperatur Aktivierungsstufe (l) | 30 | °C |
| Temperatur (Produktstufe (2) | 35 | °C |
| pH-Wert Aktivierungsstufe (l) | 4,5 | - |
| pH-Wert (Produktstufe (2) | 4,8 | - |
| Sauerstoffpartialdruck Aktivierungsstufe (l) | 3,0 | % |
| Sauerstoffpartialdruck Produktstufe (2) | 1,0 | % |

**Tabelle 2**

| resultierende Parameter (Bezeichnung laut Zeichnung l) | Wert | Einheit |
|---|---|---|
| Biomassenkonzentration Aktivierungsstufe (l) | 50 | g HTS/l |
| Biomassenkonzentration | | |
| Produktstufe (2) | 110 | g HTS/l |
| mittleres Zellalter | 65 | h |
| LNZTS Aktivierungsstufe (l) | 6,5 | % |
| LNZTS Produktstufe (2) | 10,5 | % |
| elektrische Leitfähigkeit Aktivierungsstufe (l) | 30 | mS/cm |
| elektrische Leitfähigkeit Produktstufe (2) | 50 | mS/cm |
| osmotischer Druck Aktivierungsstufe (l) | 19 | bar |
| osmotischer Druck Produktstufe (2) (jeweils ohne Äthanol gemessen) | 30 | bar |
| Äthanol Aktivierungsstufe (l) | 4,0 | Vol.% |
| Äthanol Produktstufe (2) | 6,8 | Vol.% |
| Wachstumsrate Aktivierungsstufe (l) | 0,1 | l/h |
| Wachstumsrate Produktstufe (2) | 0,006 | l/h |
| Zellaktivität Aktivierungsstufe (l) | 88 | % |
| Zellaktivität Produktstufe (2) | 80 | % |
| ZTS Aktivierungsstufe (l) | 3 | g/l |
| ZTS Produktstufe (2) | 3 | g/l |

**Tabelle 3**

| Verfahrensgrößen | Wert | Einheit |
|---|---|---|
| Produktbildung Gesamtsystem (incl. Abluftverluste) | 12,9 | l/h |
| Ausbeute (auf Theorie) (ohne Zellmassenbildung) | 99,0 | % |
| Ausbeute (auf Theorie) (incl. Zellmassenbildung) | 93,3 | % |
| Produktbildungsrate (auf aktive Gesamtbiomasse) | 0,265 | gETOH/gHTS . h |
| Raum/Zeit-Ausbeute (bezogen auf Produktfermenter (2) | 31,9 | lWTOH/m³ . h |

Es wurde festgestellt, daß nach mehreren 100 Stunden Betriebszeit die oben genannten Parameter unverändert waren.

Die ungewöhnlich hohe Raum-/Zeitausbeute von 31,9 lETOH/m³ und Stunde bei dem Rohstoff Melasse und Äthanolkonzentrationen von 6,8 Vol.% läßt sich nur mit der hier beschriebenen Prozeßführung erklären, wobei durch die gewählten Mediumsbedingungen eine Infektionszunahme nicht erlaubt wird und durch Zuführung von frischer Biomasse aus der Aktivierungsstufe 1 in die Produktstufe 2 ein hoher Aktivitätsgrad der Zellmasse erhalten wird.

In den Tabellen bedeuten:
- HTS: = Hefetrockensubstanz
- LNZTS: = lösliche Nichtzuckertrockensubstanz
- ZTS: = Zuckergehalt.

### Beispiel 2

Dieses Beispiel zeigt die Erzielung sehr hoher Raum-/Zeitausbeuten bei ebenfalls hoher Äthanolproduktkonzentration unter Einsatz eines speziellen temperatur- und osmotoleranten Hefestammes. Verwendet wurde die Hefe HETT 80, hinterlegt im Institut für Gärungsgewerbe, 1000 Berlin 65.

Die Anzucht wurde ähnlich durchgeführt wie in Beispiel 1 bei entsprechender Modifizierung der Parameter hinsichtlich des verwendeten Hefestammes.

Dieser Hefestamm weist eine hohe Temperaturtoleranz neben einer ausgeprägten Endprodukttoleranz auf. Es lassen sich somit höhere Produktbildungsraten bei belastenden Mediumsbedingungen erreichen. Allerdings sind die erzielbaren Wachstumsraten bei diesen Bedingungen sehr gering, so daß zum Erreichen des notwendigen mittleren Zellalters eine Rückführung der Biomasse aus der Produktstufe 2 in die Aktivierungsstufe 1 vorgenommen werden muß. Dies geschieht durch direkte Überführung eines Teilstromes des biomassehaltigen Retentates aus der Produktstufe 2 in die Aktivierungsstufe 1.

Die eingestellten sowie resultierenden Parameter und Verfahrensgrößen sind wiederum in den Tabellen 4, 5 und 6 wiedergegeben. Aufgrund der niedrigereren Produktkonzentration von 6,0 Vol.% Äthanol und des Einsatzes des Hefestammes HETT 80 resultiert eine verbesserte Produktbildungsrate von 0,336 g Äthanol pro g HTS . Stunde, welche zu einem Ansteigen der Raum-/Zeit-Ausbeute auf 43 l Äthanol pro m³ und Stunde führt. Die Erhaltung der Zellaktivität und ein entsprechendes Wachstum bei dieser Hefe wird durch die Reduzierung der mittleren Aufenthaltszeit der Biomasse in der Produktstufe 2 durch Rückführung eines Teilstromes in die Aktivierungsstufe 1 erreicht.

**Tabelle 4**

| wählbarer Parameter (Bezeichnung laut Zeichnung l) | Wert | Einheit |
|---|---|---|
| Arbeitsvolumen Aktivierungsstufe (l) | 80 | l |
| Arbeitsvolumen Produktstufe (2) | 400 | l |
| Zulauf Substrat (8a) | 7,2 | kg |
| Zulauf Substrat (8b) | 60 | kg |
| Produktstufenablauf (16) | 6,23 | l/h |
| Aktivierungsstufenablauf (15) | 80 | l/h |
| Produktstufenrücklauf (17) | 32,7 | l/h |
| Permeatstrom Aktivierungsstofe (13) | 10 | l/h |
| Permeatstrom Produktstufe (14) | 270 | l/h |
| Retentatstrom Aktivierungsstufe (ll) | 2 | m³/h |
| Retentatstrom Produktstufe (12) | 7 | m³/h |
| Temperatur Aktivierungsstufe (l) | 32 | °C |
| Temperatur Produktstufe (2) | 37 | °C |
| pH-Wert Aktivierungsstufe (l) | 4,5 | - |
| pH-Wert Produktstufe (2) | 5,0 | - |
| Sauerstoffpartialdruck Aktivierungsstufe (l) | 3,0 | % |
| Sauerstoffpartialdruck Produktstufe (2) | l,0 | % |

**Tabelle 5**

| resultierende Parameter (Bezeichnung laut Zeichnung l) | Wert | Einheit |
|---|---|---|
| Biomassenkonzentration Aktivierungsstufe (l) | 50 | g HTS/l |
| Biomassenkonzentration Produktstufe (2) | 110 | g HTS/l |
| mittleres Zellalter | 70 | h |
| LNZTS Aktivierungsstufe (l) | 6,0 | % |
| LNZTS Produktstufe (2) | 10,0 | % |
| elektrische Leitfähigkeit Aktivierungsstufe (l) | 26 | mS/cm |
| elektrische Leitfähigkeit Produktstufe (2) | 46 | mS/cm |
| osmotischer Druck Aktivierungsstufe (l) | 16 | bar |
| osmotischer Druck Produktstufe (2) (jeweils ohne Äthanol gemessen) | 29 | bar |
| Äthanol Aktivierungsstufe (l) | 3,8 | Vol.% |
| Äthanol Produktstufe (2) | 6,0 | Vol.% |
| Wachstumsrate Aktivierungsstufe (l) | 0,l | l/h |
| Wachstumsrate Produktstufe (2) | 0,0064 | l/h |
| Zellaktivität Aktivierungsstufe (l) | 90 | % |
| Zellaktivität Produktstufe (2) | 85 | % |
| ZTS Aktivierungsstufe (l) | 3 | g/l |
| ZTS Produktstufe (2) | 3 | g/l |

**Tabelle 6**

| Verfahrensgrößen | Wert | Einheit |
|---|---|---|
| Produktbildung Gesamtsystem (incl. Abluftverluste) | 17,2 | l/h |
| Ausbeute (auf Theorie) (ohne Zellmassenbildung) | 99,5 | % |
| Ausbeute (auf Theorie) (incl. Zellmassenbildung) | 94,8 | % |
| Produktbildungsrate (auf aktive Gesamtbiomasse) | 0,336 | gETOH/gHTS . h |
| Raum/Zeit-Ausbeute (bezogen auf Produktfermenter (2) | 43 | lETOH/m³ . h |

## Patentansprüche

1. Verfahren zur kontinuierlichen fermentativen Erzeugung von niederen aliphatischen Alkoholen oder organischen Lösungsmitteln, insbesondere von Äthanol, aus zuckerhaltigen Substraten, wie Zuckerrübenmelasse, bei dem das Substrat in zwei aufeinanderfolgenden Fermentationsstufen der Einwirkung von alkoholbildenden Mikroorganismen, insbesondere Hefe, bei unsteriler Prozeßführung ausgesetzt wird, **dadurch gekennzeichnet**, daß die erste Fermentationsstufe als Aktivierungsstufe zur Induzierung großen Zellwachstums (Zellvermehrung) und die zweite Fermentationsstufe als Produktstufe mit hoher Produktbildungsrate bei einem Volumenverhältnis der ersten zur zweiten Stufe von 1:10 bis 1:2 betrieben werden, wobei
a) 5 bis 20% der Gesamtsubstratmenge in die erste Stufe und 80 bis 95% in die zweite Stufe eingeleitet wird,
b) in die erste Stufe ein Vielfaches der in die zweite Stufe eingeleiteten Gasmenge (Sauerstoff) eingetragen und ein Sauerstoff-Partialdruck in der ersten Stufe von etwa 3% und in der zweiten Stufe von etwa 1% eingestellt wird,
c) die Temperatur der ersten Stufe auf 30 bis 35°C und der zweiten Stufe auf 35 bis 40°C eingestellt wird,
d) aus der ersten Stufe ein Teilstrom einer Mikrofiltration unterworfen und der Permeatstrom in die zweite Stufe sowie der Ablauf der ersten Stufe in die zweite Stufe geleitet wird,
e) der Ablauf der zweiten Stufe einer Mikrofiltration unterworfen und der Permeatstrom abgeführt sowie das Retentat teils als Produktstrom entnommen und teils in die zweite Stufe rückgeführt wird und
f) die Überführungsströme aus der ersten in die zweite Stufe sowie die Rückführung des Teilstromes des Retentats und der Abzug des Permeats sowie des Produktstromes aus der zweiten Stufe so eingestellt werden, daß bei Verweilzeiten der Zellmasse von 0,5 bis 2 Std. in der ersten Stufe und von 5 bis 15 Std. in der zweiten Stufe ein mittleres Zellalter von 40 bis 100 Std. erreicht wird.

2. Verfahren nach Anspruch 1, **dadurch gekennzeichnet**, daß unter Aufrechterhaltung der Bedingungen gemäß f) ein Teilstrom des Retentats aus der zweiten Stufe in die erste Stufe rückgeführt wird.

3. Verfahren nach Anspruch 1 oder 2, **dadurch gekennzeichnet**, daß in der ersten Stufe eine Mikroorganismen-Konzentration von weniger als 80 g Trockensubstanz pro l und in der zweiten Stufe ein Mikroorganismen-Konzentrat von mehr als 80 g Trockensubstanz pro l eingestellt werden.

4. Verfahren nach einem der vorhergehenden Ansprüche, **dadurch gekennzeichnet**, daß ausschließlich in die erste Stufe für das Wachstum der Mikroorganismen benötigte Wachs- und Nährstoffe eingespeist werden.

## Claims

1. A method for the continuous fermentative production of low aliphatic alcohols or organic solvents, in particular ethanol, comprising sugar-containing substrates, such as sugar-beet molasses, wherein the substrate is subjected to the action of alcohol-forming micro-organisms, in particular yeast, in two consecutive fermentation stages in non-sterile conditions, characterised in that the first fermentation stage is carried out as an activation stage for inducing a high level of cell growth (cell multiplication) and the second fermentation stage as a product stage with a high product formation rate, with a volume ratio of the first stage to the second stage of 1:10 to 1:2, wherein
a) 5% to 20% of the total substrate quantity is fed into the first stage and 80% to 95% into the second stage,
b) a multiple of the quantity of gas (oxygen) fed into the second stage is fed into the first stage and oxygen partial pressures of approximately 3% in the first stage and approximately 1% in the second stage are set,
c) the temperature of the first stage is set at 30°C to 35°C and the temperature of the second stage at 35°C to 40°C,
d) a partial flow from the first stage is subjected to micro-filtration and the permeate flow is fed into the second stage and the outflow of the first stage is fed into the second stage,
e) the outflow of the second stage is subjected to micro-filtration and the permeate flow drawn off and the residue is partly removed as a product flow and partly fed back into the second stage, and
f) the transfer flows from the first stage into the second stage and the process of feeding back the partial flow of the residue and drawing off the permeate and the product flow from the second stage are regulated such that with cell mass dwell times of 0.5 to 2 hours in the first stage and 5 to 15 hours in the second stage an average cell age of 40 to 100 hours is obtained.

2. A method according to claim 1, characterised in that a partial flow of the residue is fed back from the second stage into the first stage with retention of the conditions according to f).

3. A method according to claim 1 or 2, characterised in that in the first stage a micro-organism concentration of less than 80g dry substance per l is set and in the second stage a micro-organism concentrate of more than 80g dry substance per l is set.

4. A method according to any one of the preceding claims, characterised in that growth substances and nutrients necessary for the growth of the micro-organisms are fed exclusively into the first stage.

## Revendications

1. Procédé pour la préparation par fermentation en continu d'alcools aliphatiques inférieurs ou de solvants organiques, en particulier d'éthanol, à partir de substrats à base de sucre, tels que la mélasse de betterave à sucre, dans lequel le substrat est exposé, en deux étapes de fermentation successives, à l'effet de microorganismes alcoologènes, en particulier de la levure, le processus étant mené dans des conditions non stériles, caractérisé en ce que la première étape de fermentation est menée à titre d'étape d'activation, pour induire une grande croissance des cellules (multiplication des cellules) et la deuxième étape de fermentation est menée à titre d'étape de production, avec des taux de formation de produit élevés, dans des conditions volumétriques entre la première et la deuxième étape situés dans un rapport de 1:10 à 1:2, où
a) de 5 à 20 % de la quantité totale de substrat est introduite dans la première étape et de 80 à 95 % dans la deuxième étape,
b) il est établi dans la première étape un multiple de la quantité de gaz (oxygène) introduite dans la deuxième étape et où il est établi dans la première étape une pression partielle d'à peu près 35 % et, dans la deuxième étape, d'à peu près 1%,
c) la température de la première étape est réglée à 30 à 35°C et celle de la deuxième étape est réglée à 35 à 40°C,
d) un courant partiel provenant de la première étape est soumis à une microfiltration et le courant de perméat est dirigé dans la deuxième étape, ainsi que l'échappement de la première étape,
e) l'échappement de la deuxième étape est soumis à une microfiltration et le courant de perméat est évacué, le rétentat étant par ailleurs partiellement évacué, à titre de courant de produit, et partiellement recyclé dans la deuxième étape, et
f) les courants de transfert provenant des première et deuxièmes étape, ainsi que le recyclage du courant partiel du rétentat et l'extraction de perméat ainsi que le courant de produit provenant de la deuxième étape étant réglés de telle façon que, pour des temps de séjour de la masse cellulaire allant de 0,5 à 2 heures dans la première étape et de 5 à 15 heures dans la deuxième étape, on obtient un âge moyen de cellule situé entre 40 et 100 heures.

2. Procédé selon la revendication 1, caractérisé en ce que, tout en conservant les conditions indiquées en f), un courant partiel de rétentat provenant de la deuxième étape est recyclé dans la première étape.

3. Procédé selon la revendication 1 ou 2, caractérisé en ce que, dans la première étape, est réglée une concentration en microorganismes inférieure à 80 g de substance sèche par litre et, dans la deuxième étape, une concentration en microorganismes supérieure à 80 g de substance sèche par litre.

4. Procédé selon l'une des revendications précédentes, caractérisé en ce que les substances de croissance et nutritives nécessaires à la croissance des microorganismes sont alimentées exclusivement dans la deuxième étape.
